# EUROPEAN PATENT SPECIFICATION

(11) **EP 4 327 542 B1**
(45) Date of publication and mention of the grant of the patent: **01.04.2026**
(21) Application number: 22792327.3
(22) Date of filing: 19.04.2022
(51) Int. Cl.: H04L 67/306, G06F 3/048, G06N 20/00, G06Q 30/02, G06F 18/2135, G06F 18/23213, H04L 67/50, A61B 5/16, A61B 5/00, G06F 11/34

(54) **SYSTEMS AND METHODS TO ADAPT A DIGITAL APPLICATION ENVIRONMENT BASED ON PSYCHOLOGICAL ATTRIBUTES OF INDIVIDUAL USERS**
SYSTEME UND VERFAHREN ZUR ANPASSUNG EINER DIGITALEN ANWENDUNGSUMGEBUNG AUF BASIS PSYCHOLOGISCHER ATTRIBUTE VON INDIVIDUELLEN BENUTZERN
SYSTÈMES ET PROCÉDÉS PERMETTANT D'ADAPTER UN ENVIRONNEMENT D'APPLICATIONS NUMÉRIQUES EN FONCTION DE CARACTÉRISTIQUES PSYCHOLOGIQUES D'UTILISATEURS INDIVIDUELS

(30) Priority: 21.04.2021 US 202117236216
(43) Date of publication of application: 28.02.2024
(73) Proprietor: Solsten, Inc., Edina MN 55435 (US)
(72) Inventor: SCHAEPPI, Joseph Jack, Maple Grove, Minnesota 55311 (US)
(74) Representative: Berggren Oy
(86) International application number: PCT/US2022/025379
(87) International publication number: WO 2022/225954

(56) References cited:
- US-A1- 2007 282 912
- US-A1- 2010 179 950
- US-A1- 2012 124 062
- US-A1- 2013 111 509
- US-A1- 2014 372 344
- US-A1- 2015 094 144
- US-A1- 2017 262 164
- US-B1- 8 683 348

## Description

### FIELD OF THE DISCLOSURE

The present disclosure relates to systems and methods to adapt a digital application environment based on psychological attributes of individual users.

### BACKGROUND

It is known that within digital environments, users may be "classified" based on behaviors within the digital environments. For example, within online games, users tend to participate in various aspects of the game and eschew others. These aspects may include Player versus Player ("PvP") gameplay in which users seek to engage in competing directly with (e.g., battling) other players within the game and Player versus Environment ("PvE") gameplay in which users seek to complete "quests" or other tasks within the game to gain points, virtual items, and/or other rewards. For example, within websites, users may spend more or less time on particular pages of a website, purchase from a particular category and not others, and click on particular information, among others.

Typically, users of a digital environment are not "classified" (*e.g*., into a player type) until after they have begun interacting with the digital environment and have a large enough sample of use for classifications and/or predictions about future activities to be made based on behaviors within the environment. Much less, users are not "classified" for interactions with their own digital application environments (*e.g*., on their smartphones). Users sometimes have difficulty "finding" beneficial applications, using the beneficial application enough, and/or evading deleterious applications and/or usage thereof. Furthermore, even upon classification based on the behaviors, such classifications may not remain accurate over time. Further, such techniques fail to understand users psychologically as they interact within a digital application environment that may consequently allow the digital application environment to be uniquely adapted to a particular user.
US 2014/372344 A1 discloses that user performance and/or motivation for a computing system may be maximized by optimizing one or more target components of a user interface of the computing system.
US 2007/282912 A1 relates to a method of adapting computer programs to user profiles, including providing a user with a questionnaire to determine at least one of the user's intelligence, personality, emotional state, computer experience, sensory skills, motor skills, education, and training; compiling a user profile based on data received from the questionnaire; modifying the computer programs used by the user based on the user's profile; and storing the user profile and the computer program modifications in a database for future utilization by the user.
US 2017/262164 A1 discloses that a user interface is adjusted using a set of customization rules and displayed on a user device.
US 2015/094144 A1 discloses automatically determining a play personality of a user, wherein play personalities develop from play type and play patterns, which may be automatically determined by computing devices based on that user/subject's information and actions within a gaming environment.

### SUMMARY

The invention is defined by the independent claims. Dependent claims describe embodiments thereof. One aspect of the present disclosure relates to adapting digital application environments provided by devices associated with users. The users may be presented a psychological assessment. Based on answers provided by the users, psychological attributes may be determined for the individual users. The users may be classified into clusters of users with similar psychological attributes where the users of a given cluster use applications and interact with the digital application environment in a similar manner. Adaptations to the digital application environments may be determined for the users of the clusters based on the clusters and subsequently transmitted to the devices for implementation. As such, the psychological attributes indicated by the psychological assessment may addressed by adjusting with what and how the users interact and use their digital application environments.

One aspect of the present disclosure relates to a system configured for adapting user experience in the digital experience based on psychological attributes of individual users. The system may include one or more hardware processors configured by machine-readable instructions and electronic storage. The machine-readable instructions may include one or more instruction components. The instruction components may include one or more of usage obtainment component, information component, group component, adaptation component, and/or other instruction components.

The usage obtainment component may be configured to obtain application usage information from client computing platforms associated with users. The application usage information may characterize usage of applications within digital application environments by the users and/or other information. The client computing platforms may provide the digital application environments.

The information component may be configured to obtain stated information provided by the users. The stated information may include sets of answers to questions that relate to psychological attributes. The individual sets of answers may be provided by individual ones of the users. In some implementations, the sets of answers may include a first set of answers provided by a first user. In some implementations, the electronic storage may be configured to store, in electronic storage, information such as the stated information associated with the individual users. The information component may be configured to determine, based on the sets of answers, sets of psychological parameter values to psychological parameters for the individual users. By way of non-limiting illustration, a first set of psychological parameter values to a first set of psychological parameters may be determined for the first user.

The group component may be configured to identify clusters of users that have similar sets of psychological parameter values. The identification of the clusters may be based on the sets of psychological parameter values for the individual users. By way of non-limiting illustration, the clusters may include a first cluster that includes the first user on the basis of the first set of psychological parameter values.

The adaptation component may be configured to determine adaptations to the digital application environments, provided by the client computing platforms, for the individual users. The adaptations may be determined based on the clusters. By way of non-limiting illustration, a first adaptation to the digital application environments may be determined for the first cluster of users, including the first user. The adaptation component may be configured to transmit the adaptations to the client computing platforms for implementation. By way of non-limiting illustration, the first adaptation may be transmitted to the client computing platforms associated with the first cluster of users for implementation.

As used herein, the term "obtain" (and derivatives thereof) may include active and/or passive retrieval, determination, derivation, transfer, upload, download, submission, and/or exchange of information, and/or any combination thereof. As used herein, the term "effectuate" (and derivatives thereof) may include active and/or passive causation of any effect, both local and remote. As used herein, the term "determine" (and derivatives thereof) may include measure, calculate, compute, estimate, approximate, generate, and/or otherwise derive, and/or any combination thereof.

These and other features, and characteristics of the present technology, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of 'a', 'an', and 'the' include plural referents unless the context clearly dictates otherwise.

### BRIEF DESCRIPTION OF THE DRAWINGS

FIG. 1 illustrates a system configured to adapt a digital application environment based on psychological attributes of individual users, in accordance with one or more implementations.
FIG. 2 illustrates a method to adapt a digital application environment based on psychological attributes of individual users, in accordance with one or more implementations.
FIG. 3A illustrates an example implementation of the system configured to adapt a digital application environment based on psychological attributes of individual users, in accordance with one or more implementations.
FIG. 3B illustrates an example implementation of the system configured to adapt a digital application environment based on psychological attributes of individual users, in accordance with one or more implementations.

### DETAILED DESCRIPTION

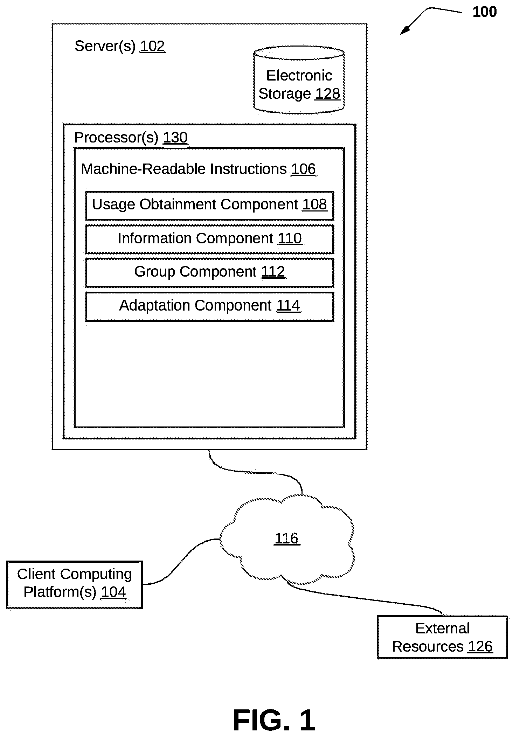

FIG. 1 illustrates a system 100 configured to adapt a digital application environment based on psychological attributes of individual users, in accordance with one or more implementations. In some implementations, system 100 may include one or more servers 102. Server(s) 102 may be configured to communicate with one or more client computing platforms 104 according to a client/server architecture and/or other architectures. Client computing platform(s) 104 may be configured to communicate with other client computing platforms via server(s) 102 and/or according to a peer-to-peer architecture and/or other architectures. Users may access system 100 via client computing platform(s) 104.

Server(s) 102 may be configured by machine-readable instructions 106. Machine-readable instructions 106 may include one or more instruction components. The instruction components may include computer program components. The instruction components may include one or more of usage obtainment component 108, information component 110, group component 112, adaptation component 114, and/or other instruction components.

Usage obtainment component 108 may be configured to obtain application usage information from client computing platforms 104 associated with users. The application usage information may characterize usage of applications within digital application environments by the users. Client computing platforms 104 may provide the digital application environments. In some implementations, the application usage information may be obtained from individual ones of the applications installed on client computing platforms 104. In some implementations, the application usage information may be obtained from the operating systems that aggregate the application usage information from the individual applications of client computing platforms 104. In some implementations, the application usage information may be obtained from a recordation application installed on client computing platforms 104 that aggregates the application usage information from the individual applications and/or other obtainment techniques.

An instance of the digital application environment may be executed by computer components to determine views (*e.g*., E-book page, content feed, shopping page, *etc.)* for individual ones of the applications within the digital application environment and the digital application environment itself. The computer components may be included in server(s) 102, client computing platforms 104, servers associated with the individual applications, or other sources. The views may then be communicated (*e.g*., via streaming, via object/position data, and/or other information) from server(s) 102 and/or sources to client computing platforms 104 for presentation to users. In some implementations, the view determined and transmitted to a given client computing platform 104 may correspond to a location in a virtual space of a gaming application or fitness application, for example, presented via the digital application environment (*e.g*., the location from which the view is taken, the location the view depicts, and/or other locations), a zoom ratio, a dimensionality of objects, a point-of-view, and/or view parameters. One or more of the view parameters may be selectable by the user. In some implementations, the views of the digital application environment may comprise icons that correspond to the applications, widgets that correspond to the applications, notifications, and/or other graphical user interface elements that facilitate the user's interaction with client computing platform 104 and the applications.

In some implementations, the instance of the digital application environment may comprise or otherwise present a simulated space that is accessible by users via clients (*e.g*., client computing platform(s) 104) that present the views of the digital application environment to a user. The simulated space may have a topography, express ongoing real-time interaction by one or more users, include one or more objects positioned within the topography that are capable of locomotion within the topography, a content feed, and/or other simulated objects. In some instances, the topography may be a 2-dimensional topography. In other instances, the topography may be a 3-dimensional topography. The topography may include dimensions of the space, and/or surface features of a surface or objects that are "native" to the space. In some instances, the topography may describe a surface (*e.g*., a ground surface) that runs through at least a substantial section of the space. In some instances, the topography may describe a volume with one or more bodies positioned therein (*e.g*., a simulation of gravity-deprived space with one or more celestial bodies positioned therein). The content feed may include various content presented as the various content is newly available (*e.g*., by the applications, by other users using the applications, *etc.).* The instance executed by the computer components may be synchronous, asynchronous, and/or semi-synchronous.

The above description of the manner in which views of the digital application environment and the simulated space are provided is not intended to be limiting. The digital application environment may be expressed in a more limited, or richer, manner. For example, views determined for the digital application environment may be selected from a limited set of graphics depicting an event in a given place within the virtual space or the simulated space. The views may include additional content (*e.g*., text, audio, pre-stored video content, and/or other content) that describes particulars of the current state of the place, beyond the relatively generic graphics. For example, a view may include a generic battle graphic with a textual description of the opponents to be confronted. For example, a view may include video content in conjunction with textual content. Other expressions of individual places within the digital application environment are contemplated.

In some implementations, within the instance(s) of the digital application environment, users may control or manipulate characters, objects, simulated physical phenomena (*e.g*., wind, rain, earthquakes, and/or other phenomena), images, videos, documents, and/or other elements within the digital application environment to interact with the digital application environment and/or other users.

The digital application environments may include a plurality of the applications. For example, the applications, and application types thereof, may include game applications, educational applications, reading application, music applications, social networking applications, entertainment applications, fitness applications, business applications, shopping applications, food & drink applications, among other applications.

The application usage information may include screen time, battery usage, Internet usage, location usage, times of installations of the individual applications, the application types of installations of the individual applications, costs of the installations, times of initiations of the individual applications, times of terminations of the individual applications, amount of the notifications, notification types of the notifications, cross-application information usage, times of in-application purchases and in-application sales, item type of the in-application purchases, the item type of the in-application sales, content types of content interacted with, interaction types of interactions, the application types of the applications initiated, and/or other application usage information.

The screen time may include durations of time the user has spent within the digital application environment via client computing platform 104 or on individual ones of the applications. The durations of time may be over a day, a week, a month, and/or other period of time. The battery usage may include a battery amount that the digital application environment depletes, a battery amount that the individual applications deplete, a battery amount that the individual applications used at a point in time or during a range of time, an amount of the battery that the digital application environment collectively used at a point in time or during a range of time, and/or other battery usage. The battery usage amount may be a proportion, a percentage, or other amount. The Internet usage may be amount of information transferred between client computing platforms 104 and the Internet during a given period of time (*e.g*., an hour, a day, a week, a month, *etc.).* The information may be in bytes, kilobytes, megabytes, gigabytes, or other measurement of information transfer. The location usage may include the applications that use or have permission to use location information from client computing platforms 104, an amount of time that the location information is used, or other location usage. The location information may include a determination of a real-world position or geographic location of the user based on one or more of signal strength, GPS, cell tower triangulation, Wi-Fi location, receipt of GPS coordinates or an address, and/or other location information. In some implementations, user movements may be tracked using a geographybased transmitter on client computing platforms 104.

The times of installations of the applications may indicate installation frequency over the given period of time. The application types of the installations may indicate which application type is most frequently installed or used by the users and/or other information. The costs of the installations may indicate an amount of currency (*e.g*., US Dollars, virtual currency, credits, *etc.)* that the user spends for the individual applications, for the applications of the application types, other costs related to the installations, and/or other information.

The times of initiations of the applications may refer to a time and/or date when the individual applications are initiation or opened. In some implementations, the times of the initiations may indicate frequency of use of the individual applications and/or other information. The times of the terminations of the applications may refer to a time and/or a date when the individual applications are terminated or closed. In some implementations, the times of the terminations and the times of the initiations may indicate an amount of time spent by the user on or interacting with the individual applications, all the applications, and/or other information.

The amount of the notifications may include an amount of notifications received by the users via client computing platforms 104 from all the applications within the period of time, an amount of notifications received by the users via client computing platforms 104 from the individual applications, a time and/or date of the individual notifications, and/or other information related to notifications. The notification types of the notifications may include a warning notification, an informative notification, a promotional notification, a reminder notification, and/or other notification types. In some implementations, the notification types may include the application type the notification originated from and/or other information. The warning notification may warn the user with regard to their mental health, physical health, psychological parameter values, and/or other information. The informative notification may inform the user of various information related to the individual applications (*e.g*., a weather application). The promotional notification may inform the user about promotions, coupons, sales, availability, and/or other information related to purchasable or sellable items (*e.g*., within the shopping applications or other applications). The reminder notification may remind the user of suggestions and/or other reminders. In some implementations, the notifications may be based on the other application usage information or may be from the individual applications.

The cross-application information usage may include which of the applications use information from other ones of the applications. For example, a social networking application may use search history from the shopping applications (*e.g*., to provide a promotional notification). The times of the in-application purchases and the in-application sales may be a date and/or time at which a purchase or a sale was made within the applications. For example, a date and time of a purchase of extra points within a game application may be included in the application usage information. The item type of the in-application purchase may include a subscription, a virtual item (*e.g*., a virtual sword to be used in a game within the game application, virtual currency (*e.g*., points), a level-up in the game, *etc.),* a real-world item (*e.g*., shoes via the shopping application), and/or other item types of virtual or real-world items that may be purchased via the applications. The item type of the in-application sale may include the same item types of the in-application purchases.

The content types of the content interacted with may include a game, a picture, a short video, a long video, feedback questions, advertisements, audio (*e.g*., music, podcast, audiobook), E-books, news, comments, purchasable items, and/or other content types. The interaction types of the interactions may include interactions with content, interactions with other users, and/or other interaction types. The interactions with other ones of the users may include one or more calls, text messages, instant messages, in-application messages, video call, and/or other interactions with the users. The interactions may be by the users via their respective client computing platforms 104. The interactions may be routed to and from the appropriate users through server(s) 102, the Internet, and/or based on Internet and communication service providers (*e.g*., phone plan). The interactions with the content may include one or more of liking of the content, sharing of the content, watching of the content (*e.g*., a short video, a long video, a live stream, *etc.),* viewing of the content (*e.g*., viewed an image), saving of the content, and/or other interactions with the content. The application usage information described herein are exemplary and are not intended to be limiting as other application usage information is comtemplated.

Information component 110 may be configured to obtain stated information provided by the users. The stated information may include sets of answers to questions that relate to psychological attributes. The questions that are related to the psychological attributes may be predefined by an administrator of system 100 and/or modifiable by the administrator of system 100. In some implementations, information component 110 may be configured to select a set of the questions to be presented to the users for answers. In some implementations, selecting the set of the questions may be random and based on an amount of questions (*e.g*., randomly pick 10 questions). The amount of questions to randomly select may be predefined by the administrator and/or modifiable by the administrator. In some implementations, the set of questions may be the same fixed questions (*e.g*., the same 15 questions are presented to all the users). The individual sets of answers may be provided by individual ones of the users. In some implementations, usage obtainment component 108 may be configured to effectuate presentation of the questions via graphical user interfaces of client computing platforms 104 or via other platforms. The presentation may be effectuated responsive events such as a first initiation of client computing platform 104 (*e.g*., a new smartphone), responsive to receipt of user input indicating to present the questions (*e.g*., opting-in to providing the stated information), and/or other events. The sets of answers may be transmitted via a network 116 to the one or more processors 130 (*i.e*., information component 110).

By way of non-limiting illustration, the sets of answers may include a first set of answers provided by a first user. In some implementations, information component 110 may be configured to obtain the stated information that includes a second set of answers provided by a second user and/or other sets of answers provided by other users. Thus, the set of answers may include the first set of answers, the second set of answers, and/or the other sets of answers. The stated information provided by the users may be stored to electronic storage 128.

In some implementations, information component 110 may be configured to determine behavioral information of the users. The behavioral information may characterize performances of behavior patterns related to the usage of the applications by the users within the digital application environment. By way of non-limiting example, the behavior patterns may include individual actions, sets of the actions, ordered sets of the actions, or multiple of the actions performed by the users. The actions may include the in-application purchase, the in-application sale, installations of the applications, purchases of the applications, the interactions with users, the interactions with content, initiations of particular ones of the applications, terminations of the particular applications, and/or other actions. In some implementations, the determined behavioral information may be stored to electronic storage 128. In some implementations, the behavioral information may be obtained from the operating systems that aggregate the behavioral information from the individual applications of client computing platforms 104. In some implementations, the behavioral information may be obtained from a recordation application installed on client computing platforms 104 that aggregates the behavioral information from the individual applications and/or other obtainment techniques. In some implementations, the behavioral information may be obtained and/or determined in the same manner as the application usage information. In some implementations, the behavioral information may be obtained in an ongoing manner. The term "ongoing manner" as used herein may refer to continuing to perform an action (e.g., determine) periodically (e.g., every 30 seconds, every minute, every hour, etc.) until receipt of an indication to terminate. The indication to terminate may include powering off client computing platform 104, charging one or more of a battery of client computing platform 104, resetting client computing platform 104, receipt of user input, and/or other indications of termination.

Information component 110 may be configured to determine sets of psychological parameter values to psychological parameters for the individual users. The determination of the sets of psychological parameter values may be based on the sets of answers provided by the individual users. As a result, for example, a first set of psychological parameter values to psychological parameters may be determined for the first user, a second set of psychological parameter values for the second user, and/or other sets of psychological parameter values may be determined for other users. The first set may be based on the first set of answers provided by the first user. The second set may be based on the second set of answers provided by the second user.

The psychological parameter values may characterize, by way of non-limiting example, achievement motivation, motivation, personality inventory, cultural values, competitiveness, positive and negative affect before, during, and/or after engagement with the digital experience (*i.e*., emotions), communication style, personal values, daily routines/activities, life/gaming pain points, life/gaming hopes and aspirations, wellbeing, user experience, gaming/experience using time, subscription behavior, affinity information, personality, emotional style, goal orientation, goal commitment, ego and task orientation, relatedness, sense of community, social influence, social identity, group identification, we-identity, quality of life, satisfaction with life, work-related quality of life, mindfulness, happiness, emotional intelligence, self-awareness/internal awareness, external awareness, connectedness to nature, social connectedness, social bonding, perceived stress, depression, anxiety, decision-making style, thinking style, critical thinking, cognitive approach to learning, learning style, attributional style, internality-externality, stabilityinstability, global-specific, creativity, curiosity, playfulness, exploration, mental strength, grit, flourishing, gratitude, inspiration, spirituality, hedonism, materialism/material values, perceptions, sentiments, and/or other psychological parameters.

Achievement motivation may include compensatory effort, competitiveness, confidence in success, dominance, eagerness to learn, engagement, fearlessness, flexibility, flow, goal setting, independence, internality, persistence, preference in difficult tasks, pride in productivity, self-control, status orientation, ambition, self-assurance, and/or other psychological parameters. Motivation may include mastery, purpose, autonomy, and/or other psychological parameters.

Personality inventory may include neuroticism, openness, conscientiousness, extraversion, and agreeableness and/or other psychological parameters. Neuroticism may include anxiety, impulsiveness, vulnerability, and/or other psychological parameters. Openness may include fantasy, feelings/empathy, action, and/or other psychological parameters. Conscientiousness may include achievement striving, competence, self-discipline, and/or other psychological parameters. Extraversion may include warmth assertiveness, activity, and/or other psychological parameters. Agreeableness may include trust, altruism, modesty, and/or other psychological parameters.

Cultural values may include individualism, indulgence, long term orientation, masculinity, power distance, uncertainty avoidance, and/or other psychological parameters. Competitiveness may include avoidant, collaborative, competitive affectivity, dependent, dominant, general competitiveness, independent, personal enhancement, and/or other psychological parameters.

Positive and negative affect before, during, and/or after engaging in the digital experience may include hostility, joviality, negative emotions, positive emotions, sadness, self-assurance, and/or other psychological parameters. Communication style may include feeler, intuitor, sensor, thinker, and/or other psychological parameters.

Wellbeing may include social wellbeing, psychological wellbeing, physical wellbeing, physical activity, sleep, bounded reciprocity, resilience grit, and/or other psychological parameters.

Personality may include anger, hostility, depression, selfconscientiousness, excitement-seeking, positive emotions, gregariousness, ideas, values, aesthetics, tendermindedness, straightforwardness, compliance, deliberation, order, dutifulness, and/or other psychological parameters.

Emotional style may include resilience, outlook, social intuition, self-awareness, sensitivity to context, attention, and/or other psychological parameters.

Goal orientation may include mastery approach/learning goal orientation, performance approach/performance goal orientation, performance avoid/avoidance goal orientation, and/or other psychological parameters.

Work-related quality of life may include structure, boundaries, focus, efficiency, information provision, communication, psychological support, stress at/from work, psychological safety, connectedness with team, motivation to work, adaptability, job/career satisfaction, control at work, home-work interface, general wellbeing, working conditions, and/or other psychological parameters.

Mindfulness may include observing, describing, acting with awareness, non-judgment, non-reactivity, and/or other psychological parameters.

Emotional intelligence may include emotion perception, emotion expression, emotion management, emotion regulation, impulse control, relationships, stress management, and/or other psychological parameters.

Social connectedness may include social connectedness, loneliness, membership self-esteem, private self-esteem, public self-esteem identity self-esteem, interdependent self, independent self, social avoidance, social distress, and/or other psychological parameters.

Decision-making style may include respected, confident, spontaneous, dependent, vigilant, avoidant, brooding, intuitive, anxious, and/or other psychological parameters.

Thinking style may include intuitive, experiential, analytical, rational, and/or other psychological parameters.

Cognitive approaches to learning may include avoidant, participative, competitive, collaborative, dependent, independent, and/or other psychological parameters.

Learning style may include visual (spatial), aural (auditory-musical), verbal (linguistic), physical (kinesthetic), logical (mathematical), social (interpersonal), solitary (intrapersonal), and/or other psychological parameters.

Mental strength may include tenacity, confidence, optimism, adaptability, self-awareness, reliability, responsibility, well-being, and/or other psychological parameters.

Flourishing may include positive emotion, engagement, relationships, meaning, accomplishment, health, loneliness, and/or other psychological parameters.

By way of non-limiting example, the psychological parameter values to the psychological parameters may be a number score on a predetermined range unique to each psychological parameter, a letter score, and/or other type of value than may characterize a particular user as whole.

In some implementations, information component 110 may be configured to store, in electronic storage 128, information (*e.g.*, the sets of answers, the psychological parameter values) associated with the individual users. In some implementations, the determined psychological parameter values for individual users may be communicated with entities (*e.g*., game companies, website developers, user experience companies, device companies, *etc.).* The entities may download, export, purchase, subscribe to, obtain in real-time, and/or other obtainments the determined psychological parameter values for individual users.

Group component 112 may be configured to identify clusters of users that have similar sets of the psychological parameter values. The identification of clusters may be based on the sets of psychological parameter values for the individual users and/or other information. The identifying of the clusters of the users may include, by way of non-limiting example, latent class analysis, hierarchical clustering, k-means clustering, mean-shifting clustering, machine learning, dimensionality reduction, principal component analysis, supervised learning, and/or other grouping techniques. The users may be classified into clusters of users with similar sets of psychological parameter values. That is, given the similar sets of psychological parameter values, the users in a given cluster may interact with digital application environments that include the same applications or similar application types, and/or use the same applications or the applications of the similar application type and the digital application environments in a similar manner. The clusters may include a first cluster, a second cluster, and/or other clusters. The first cluster may include the first user on the basis of the first set of psychological parameter values. The second cluster may include other users that have other similar sets of psychological parameter values. The second cluster may include the second user on the basis of the second set of psychological parameter values.

In some implementations, group component 112 may be configured to identify the clusters of the users based on the behavioral information, the sets of psychological parameter values, and/or other information. In some implementations, the users may be included in multiple clusters or the cluster the individual users are included in may change based on the behavioral information. In some implementations, the determined behavioral information may be stored to electronic storage 128 in association with the users.

Adaptation component 114 may be configured to determine adaptations to the digital application environment provided by client computing platforms 104 for the individual users based on the clusters. In some implementations, the adaptations may be based on the behavioral information. The behavioral information may include at least two of the behavior patterns of which the adapting of the digital application environment (*i.e*., the adaptations) is based on. By way of non-limiting illustration, a first adaptation to the digital application environment may be determined for the first cluster of users, including the first user. A second adaptation to the digital application environment may be determined for the second cluster of users, including the second user. The adaptations to the digital application environment may be modifications to user experiences for the users of the individual clusters. Modifications to the user application environment may result in a plurality of variations of the digital application environment.

For example, based on the first cluster that the first user is included in, some of the applications or applications of the same application type of the digital application environment for the first user (and the digital application environments of the users of the first cluster) may be temporarily restricted from accessing by the users. For example, based on the determined psychological parameter values of a particular user, the digital application environments may be modified to be unique to the particular user. For example, based on the second cluster that the second user is included in, some of the applications within the digital application environment may be more prominent than other, such as the reading applications are more prominent than the social networking applications.

The adaptations to the digital application environment may be modifications to the appearance and/or aesthetic of the digital application environment and/or the applications thereof. For example, based on the second cluster that the second user is included in, the brightness, colors, size, layout, landscape, animations, fonts, font sizes, shapes, user interface elements, and/or other user interface presentations, and/or other appearances of the digital application environment may be modified. User interface elements may be configured to facilitate user interaction with a user interface, user entry, and/or selection. By way of non-limiting illustration, the user interface elements may include one or more of text input fields, drop down menus, check boxes, display windows, virtual buttons, and/or other user interface elements. In some implementations, the adaptations may be are designed to enhance prospective usage of the digital application environment by the users (e.g., determined beneficial application based on the psychological parameter values, the application usage information, etc.), cause less usage of the digital application environment (e.g., determined deleterious application based on the psychological parameter values, the application usage information, etc.), facilitate usage of particular ones of the applications, facilitate less usage of other particular ones of the applications, and/or other causes.

By way of non-limiting example, the adaptations may include one or more of presenting one or more of the notifications via graphical user interfaces of client computing platforms 104, restricting access to particular ones of the applications, moving the icons that initiate the applications upon selection on the graphical user interfaces, omitting the icons from application suggestions, terminating particular ones of the applications after a particular amount of time, permitting access to the particular applications after a predetermined amount of time elapses, setting a timer for the amount of time to elapse, and/or other adaptations. The notification may include a recommendation, a suggestion, and/or other notifications. The recommendations may include actions, determined based on their cluster, that the user is advised to do (*e.g*., recommend the user to use or interact with, or not, specific ones of the applications or applications of a specific application type). The suggestion may include particular ideas, plans, and/or strategies for the application usage by the user to consider executing, following, and/or is determined they will enjoy.

The adaptations may include adjusting, adjusting a difficulty setting for and/or game content within the gaming applications, means of communication that facilitate the interactions with other users, an offer to sell one or more virtual items made available, and/or other adaptations.

The adjusted difficulty setting may adjust how challenging one or more aspects of the digital experience are. By way of non-limiting example, adjusting difficulty settings may include adjusting how challenging a digital experience (*e.g.*, online game) as a whole is, how challenging particular tasks are (*e.g*., building, battling, problem solving, *etc.),* how challenging it is to complete one or more levels, adjusting how challenging it is to complete a level with every advancement of a level, and/or other difficulty setting adjustments.

The game content may be made available or omitted within the gaming applications. Such game content (or pieces thereof) may include one or more of a player-controlled character, a non-player-controlled character, a task, a quest, an assignment, a mission, a level, a chapter, a mini-game, a virtual item, a virtual resource (*e.g*., weapon, tool), of in-game powers, in-game skills, in-game technologies, and/or other pieces of game content. By way of non-limiting example, virtual items may include one or more of clothing, pets, transportation units (*e.g*., aircrafts, motor vehicles, watercrafts, *etc.),* units, buildings, and/or other virtual items.

Adjusting the means of communication may include adjusting the means of communication made available to be used contemporaneously, made available to be used one at a time, omitted, and/or other adjustments on means of communication. The means of communications may include communication applications. The applications may include the communication applications. For example, the communication applications may be native to operating systems of the client computing platforms 104 or installed. In some implementations, adjusting the means of communication may include adjusting when the interactions are sent, when the interactions are received, to whom the interactions are sent, from whom the interactions are received, and/or other adjustments to the means of communication.

In some implementations, adaptations to the digital application environments may be made for individual users without the individual users being grouped into a cluster. Thus, such adaptations may be unique to the particular user.

Adaptation component 114 may be configured to transmit the adaptations to client computing platforms 104 for implementation. By way of non-limiting illustration, the first adaptation may be transmitted to client computing platforms 104 associated with the first cluster of users for implementation. In some implementations, the adaptations may be implemented immediately. In some implementations, the adaptations may be implemented during a particular time of day (*e.g*., during sleep hours of 12AM-4AM). In some implementations, the adaptations may be implemented responsive to user input that approves of the adaptations. In some implementations, upon multiple adaptations being transmitted to client computing platforms 104, some of the multiple adaptations may be approved via the user input and some of the multiple adaptations may be denied via the user input. In some implementations, the user input may be received (by adapation component 114) from client computing platforms 104 associated with the users and/or from client computing platforms 104 associated with caregivers of the users. For example, the caregivers may include a parent, a guardian, a doctor, a therapist, a teacher, other healthcare providers, and/or other caregivers.

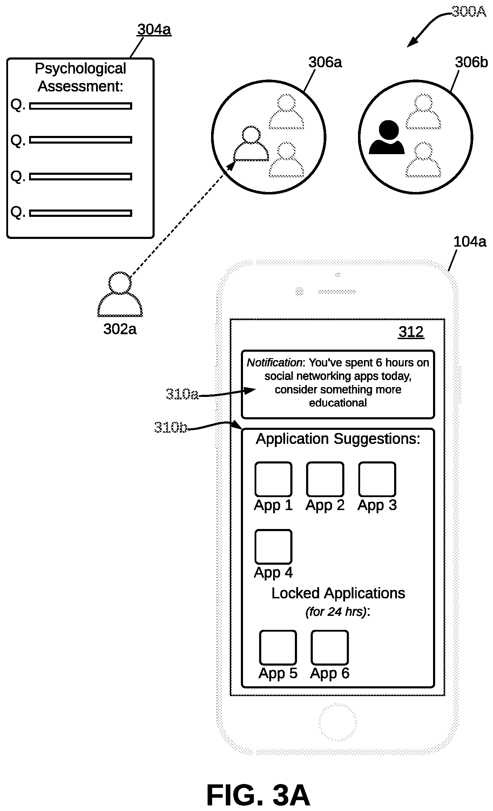

FIG. 3A illustrates an example implementation to adapt a digital application environment based on psychological attributes of individual users, in accordance with one or more implementations. FIG. 3A illustrates adaptation implementation 300A. Adaptation implementation 300A includes a question set 304a that may be presented to a user 302a. Answers provided by user 302a may be determinative of psychological parameter values to psychological parameters (as described in FIG. 1). Based on the psychological parameter values, user 302a may be included in a cluster 306a of users with similar psychological parameter values instead of a cluster 306b. Based on the similar psychological parameter values of cluster 306a, it may be determined that the users, including user 302a, of cluster 306a has social distress and visual (spatial) and aural (auditory-musical) learning styles. Thus, adaptations 310a and 310b to a digital application environment 312 provided by a client computing platform 104a associated with user 302a may be determined and transmitted to client computing platform 104a for implementation. Adaptation 310a may be a notification, particularly a suggestion related to application usage. Adaptation 310b may be presentation of suggested applications (*e.g*., App 1-4, visual and auditory-musical educational applications) and applications with restricted access (*e.g*., App 5-6, social networking applications).

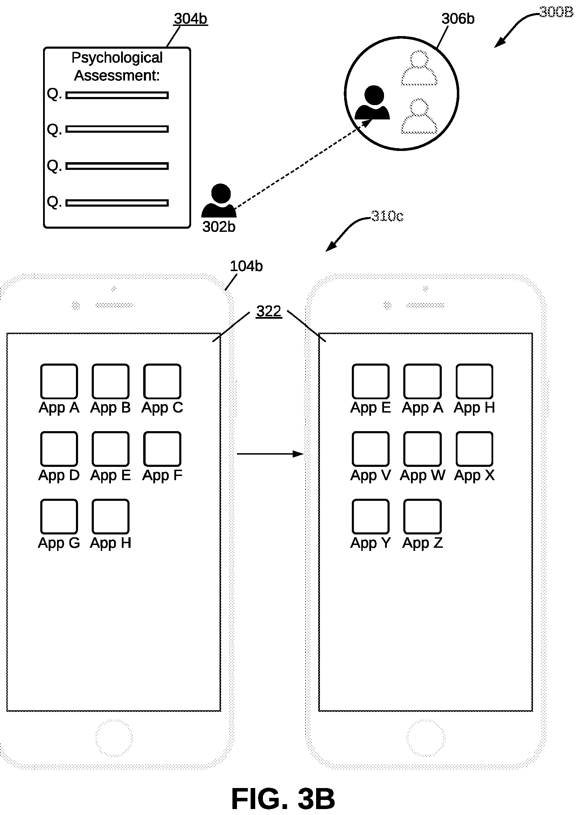

FIG. 3B illustrates an example implementation to adapt a digital application environment based on psychological attributes of individual users, in accordance with one or more implementations. FIG. 3B illustrates adaptation implementation 300B. Adaptation implementation 300B includes a question set 304b that may be presented to a user 302b. Question set 304b may be the same as or similar to question set 302a presented to user 302a of FIG. 3A. Similar to the answers provided by user 302a of FIG. 3A, answers provided by user 302b may be determinative of psychological parameter values. Based on the psychological parameter values, user 302b may be included in cluster 306b of users with similar psychological parameter values instead of cluster 306a (in FIG. 3A) with user 302a. Based on the similar psychological parameter values of cluster 306b, it may be determined that the users, including user 302b, of cluster 306b are creative, enjoy social connectedness, and are lonely. Thus, an adaptation 310c to a digital application environment 322 provided by a client computing platform 104b associated with user 302b may be determined and transmitted to client computing platform 104b for implementation. Adaptation 310c may be a rearrangement of the applications included in digital application environment 322 so that App E, A, H, and V-Z are presented instead of App A-H on a home screen of digital application environment 322.

In some implementations, server(s) 102, client computing platform(s) 104, and/or external resources 126 may be operatively linked via one or more electronic communication links. For example, such electronic communication links may be established, at least in part, via a network such as the Internet and/or other networks. It will be appreciated that this is not intended to be limiting, and that the scope of this disclosure includes implementations in which server(s) 102, client computing platform(s) 104, and/or external resources 126 may be operatively linked via some other communication media.

A given client computing platform 104 may include one or more processors configured to execute computer program components. The computer program components may be configured to enable an expert or user associated with the given client computing platform 104 to interface with system 100 and/or external resources 126, and/or provide other functionality attributed herein to client computing platform(s) 104. By way of non-limiting example, the given client computing platform 104 may include one or more of a desktop computer, a laptop computer, a handheld computer, a tablet computing platform, a NetBook, a Smartphone, a gaming console, and/or other computing platforms.

External resources 126 may include sources of information outside of system 100, external entities participating with system 100, and/or other resources. In some implementations, some or all of the functionality attributed herein to external resources 126 may be provided by resources included in system 100.

Server(s) 102 may include electronic storage 128, one or more processors 130, and/or other components. Server(s) 102 may include communication lines, or ports to enable the exchange of information with a network and/or other computing platforms. Illustration of server(s) 102 in FIG. 1 is not intended to be limiting. Server(s) 102 may include a plurality of hardware, software, and/or firmware components operating together to provide the functionality attributed herein to server(s) 102. For example, server(s) 102 may be implemented by a cloud of computing platforms operating together as server(s) 102.

Electronic storage 128 may comprise non-transitory storage media that electronically stores information. The electronic storage media of electronic storage 128 may include one or both of system storage that is provided integrally (*i.e*., substantially non-removable) with server(s) 102 and/or removable storage that is removably connectable to server(s) 102 via, for example, a port (*e.g*., a USB port, a firewire port, *etc.)* or a drive (*e.g*., a disk drive, *etc.).* Electronic storage 128 may include one or more of optically readable storage media (*e.g*., optical disks, *etc.),* magnetically readable storage media (*e.g*., magnetic tape, magnetic hard drive, floppy drive, *etc.),* electrical charge-based storage media (*e.g.,* EEPROM, RAM, *etc.),* solid-state storage media (*e.g*., flash drive, *etc.),* and/or other electronically readable storage media. Electronic storage 128 may include one or more virtual storage resources (*e.g*., cloud storage, a virtual private network, and/or other virtual storage resources). Electronic storage 128 may store software algorithms, information determined by processor(s) 130, information received from server(s) 102, information received from client computing platform(s) 104, and/or other information that enables server(s) 102 to function as described herein.

Processor(s) 130 may be configured to provide information processing capabilities in server(s) 102. As such, processor(s) 130 may include one or more of a digital processor, an analog processor, a digital circuit designed to process information, an analog circuit designed to process information, a state machine, and/or other mechanisms for electronically processing information. Although processor(s) 130 is shown in FIG. 1 as a single entity, this is for illustrative purposes only. In some implementations, processor(s) 130 may include a plurality of processing units. These processing units may be physically located within the same device, or processor(s) 130 may represent processing functionality of a plurality of devices operating in coordination. Processor(s) 130 may be configured to execute components 108, 110, 112, and/or 114, and/or other components. Processor(s) 130 may be configured to execute components 108, 110, 112, and/or 114, and/or other components by software; hardware; firmware; some combination of software, hardware, and/or firmware; and/or other mechanisms for configuring processing capabilities on processor(s) 130. As used herein, the term "component" may refer to any component or set of components that perform the functionality attributed to the component. This may include one or more physical processors during execution of processor readable instructions, the processor readable instructions, circuitry, hardware, storage media, or any other components.

It should be appreciated that although components 108, 110, 112, and/or 114 are illustrated in FIG. 1 as being implemented within a single processing unit, in implementations in which processor(s) 130 includes multiple processing units, one or more of components 108, 110, 112, and/or 114 may be implemented remotely from the other components. The description of the functionality provided by the different components 108, 110, 112, and/or 114 described below is for illustrative purposes, and is not intended to be limiting, as any of components 108, 110, 112, and/or 114 may provide more or less functionality than is described. For example, one or more of components 108, 110, 112, and/or 114 may be eliminated, and some or all of its functionality may be provided by other ones of components 108, 110, 112, and/or 114. As another example, processor(s) 130 may be configured to execute one or more additional components that may perform some or all of the functionality attributed below to one of components 108, 110, 112, and/or 114.

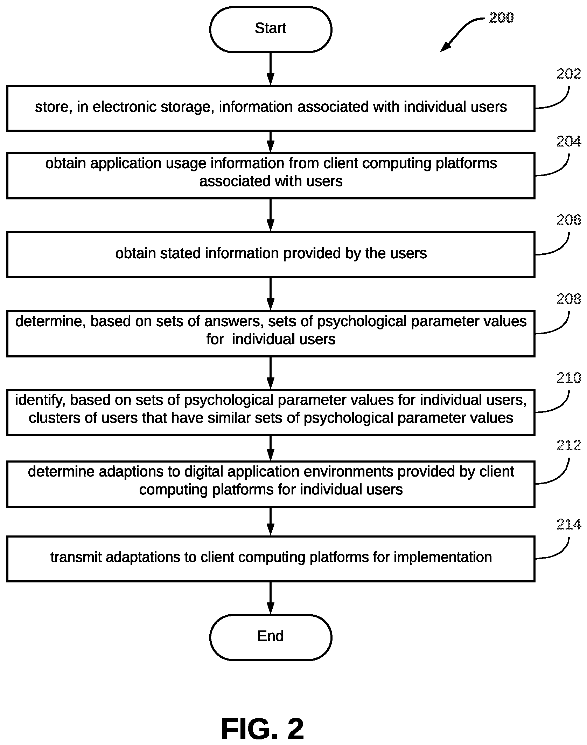

FIG. 2 illustrates a method 200 to adapt a digital application environment based on psychological attributes of individual users, in accordance with one or more implementations. The operations of method 200 presented below are intended to be illustrative. In some implementations, method 200 may be accomplished with one or more additional operations not described, and/or without one or more of the operations discussed. Additionally, the order in which the operations of method 200 are illustrated in FIG. 2 and described below is not intended to be limiting.

In some implementations, method 200 may be implemented in one or more processing devices (*e.g*., a digital processor, an analog processor, a digital circuit designed to process information, an analog circuit designed to process information, a state machine, and/or other mechanisms for electronically processing information). The one or more processing devices may include one or more devices executing some or all of the operations of method 200 in response to instructions stored electronically on an electronic storage medium. The one or more processing devices may include one or more devices configured through hardware, firmware, and/or software to be specifically designed for execution of one or more of the operations of method 200.

An operation 202 may include storing, in electronic storage, information associated with the individual users. Operation 202 may be performed by one or more hardware processors configured by machine-readable instructions including a component that is the same as or similar to information component 110 and electronic storage 128, in accordance with one or more implementations.

An operation 204 may include obtaining application usage information from client computing platforms associated with users. The application usage information may characterize usage of applications within digital application environments by the users. The client computing platforms may provide the digital application environments. Operation 204 may be performed by one or more hardware processors configured by machine-readable instructions including a component that is the same as or similar to usage obtainment component 108, in accordance with one or more implementations.

An operation 206 may include obtaining stated information provided by the users. The stated information may include sets of answers to questions that relate to psychological attributes. The individual sets of answers may be provided by individual ones of the users. Operation 206 may be performed by one or more hardware processors configured by machine-readable instructions including a component that is the same as or similar to information component 110, in accordance with one or more implementations.

An operation 208 may include determining, based on the sets of answers, sets of psychological parameter values for the individual users. Operation 208 may be performed by one or more hardware processors configured by machine-readable instructions including a component that is the same as or similar to information component 110, in accordance with one or more implementations.

An operation 210 may include identifying, based on the sets of psychological parameter values for the individual users, clusters of users that have similar sets of psychological parameter values. Operation 210 may be performed by one or more hardware processors configured by machine-readable instructions including a component that is the same as or similar to group component 112, in accordance with one or more implementations.

An operation 212 may include determining adaptations to the digital application environments provided by the client computing platforms for the individual users. The determination of the adaptations may be based on the clusters. Operation 212 may be performed by one or more hardware processors configured by machine-readable instructions including a component that is the same as or similar to adaptation component 114, in accordance with one or more implementations.

An operation 214 may include transmitting the adaptations to the client computing platforms for implementation. Operation 214 may be performed by one or more hardware processors configured by machine-readable instructions including a component that is the same as or similar to adaptation component 114, in accordance with one or more implementations.

Although the present technology has been described in detail for the purpose of illustration based on what is currently considered to be the most practical and preferred implementations, it is to be understood that such detail is solely for that purpose and that the technology is not limited to the disclosed implementations. For example, it is to be understood that the present technology contemplates that, to the extent possible, one or more features of any implementation can be combined with one or more features of any other implementation.

## Claims

1. A system (100) configured to adapt a digital application environment (312, 322) based on psychological attributes of individual users, the system comprising:
electronic storage (128) configured to store information associated with the individual users;
one or more processors (130) configured by machine-readable instructions (106) to:
obtain (204) application usage information from client computing platforms (104a, 104b) associated with users, wherein the application usage information characterizes usage of applications within digital application environments (312, 322) by the users, wherein the client computing platforms (104a, 104b) provide the digital application environments (312, 322);
obtain (206) stated information provided by the users, wherein the stated information includes sets of answers to questions that relate to psychological attributes, wherein the individual sets of answers are provided by individual ones of the users such that the sets of answers include a first set of answers provided by a first user;
determine (208), based on the sets of answers, sets of psychological parameter values to psychological parameters for the individual users such that a first set of psychological parameter values to a first set of psychological parameters is determined for the first user;
identify (210), based on the application usage information and the sets of psychological parameter values for the individual users, clusters (306a, 306b) of users that have similar sets of psychological parameter values, the clusters (306a, 306b) including a first cluster that includes the first user on the basis of the first set of psychological parameter values;
determine (212) adaptations (310a, 310b, 310c) to the digital application environments (312, 322) provided by the client computing platforms (104a, 104b) for the individual users based on the clusters (306a, 306b) such that a first adaptation to the digital application environments (312, 322) is determined for the first cluster of users, including the first user; and
transmit (214) the adaptations (310a, 310b, 310c) to the client computing platforms (104a, 104b) for implementation such that the first adaptation is transmitted to the client computing platforms (104a, 104b) associated with the first cluster of users for implementation,
**characterised in that**
the adaptations (310a, 310b, 310c) include restricting access to particular ones of the applications, moving icons that initiate the applications upon selection on the graphical user interfaces, omitting the icons from application suggestions, terminating particular applications after a particular amount of time, and/or permitting access to the particular applications after a predetermined amount of time elapses.

2. The system (100) of claim 1, wherein the adaptations (310a, 310b, 310c) further include presenting a notification (310a) via graphical user interfaces of the client computing platforms (104a, 104b), wherein the notification includes a recommendation and/or a suggestion (310b).

3. The system (100) of claim 1, wherein the psychological parameter values characterize motivations, emotions, emotional intelligence, cultural values, personal values, communication style, personality, psychological wellbeing, and/or learning style.

4. The system (100) of claim 1, wherein identifying the clusters (306a, 306b) of the users includes latent class analysis, hierarchical clustering, K-means clustering, mean-shifting clustering, machine learning, dimensionality reduction, and/or principal component analysis.

5. The system (100) of claim 1, wherein the one or more processors (130) are further configured by machine-readable instructions (106) to:
determine behavioral information of the users, wherein identifying the clusters (306a, 306b) of the users is based on the behavioral information,
wherein the behavioral information characterizes performances of behavior patterns related to the usage of the applications by the users within the digital application environment (312, 322), wherein the behavior patterns include individual actions, sets of the actions, ordered sets of the actions, or multiple of the actions performed by the users, wherein the actions include an in-application purchase, an in-application sale, installations of applications, purchases of applications, interactions with users, interactions with content, initiations of particular applications, and/or terminations of the particular applications.

6. The system (100) of claim 5, wherein the application usage information includes screen time, battery usage, Internet usage, location usage, times of the installations, application types of the installations, costs of the installations, times of the initiations, times of the terminations, amount of notifications, notification types of the notifications, cross-application information usage, times of the in-application purchases and the in-application sales, item type of the in-application purchases, the item type of the in-application sales, content types of the content interacted with, interaction types of the interactions, and/or the application types of the applications initiated.

7. The system (100) of claim 1, wherein the application usage information is obtained from individual ones of the applications installed on the client computing platforms (104a, 104b), and/or from a recordation application installed on the client computing platforms (104a, 104b) that aggregates the application usage information from the individual applications.

8. A method (200) to adapt a digital application environment (312, 322) based on psychological attributes of individual users, the method comprising:
storing (202), in electronic storage (128), information associated with the individual users;
obtaining (204) application usage information from client computing platforms (104a, 104b) associated with users, wherein the application usage information characterizes usage of applications within digital application environments (312, 322) by the users, wherein the client computing platforms (104a, 104b) provide the digital application environments (312, 322) ;
obtaining (206) stated information provided by the users, wherein the stated information includes sets of answers to questions that relate to psychological attributes, wherein the individual sets of answers are provided by individual ones of the users such that the sets of answers include a first set of answers provided by a first user;
determining (208), based on the application usage information and the stated information, sets of psychological parameter values to psychological parameters for the individual users such that a first set of psychological parameter values to a first set of psychological parameters is determined for the first user;
identifying (210), based on the application usage information and the sets of psychological parameter values for the individual users, clusters (306a, 306b) of users that have similar sets of psychological parameter values, the clusters including a first cluster that includes the first user on the basis of the first set of psychological parameter values;
determining (212) adaptations (310a, 310b, 310c) to the digital application environments (312, 322) provided by the client computing platforms (104a, 104b) for the individual users based on the clusters (306a, 306b) such that a first adaptation to the digital application environments (312, 322) is determined for the first cluster of users, including the first user; and
transmitting (214) the adaptations (310a, 310b, 310c) to the client computing platforms (104a, 104b) for implementation such that the first adaptation is transmitted to the client computing platforms (104a, 104b) associated with the first cluster of users for implementation,
**characterised in that**
the adaptations (310a, 310b, 310c) include restricting access to particular ones of the applications, moving icons that initiate the applications upon selection on the graphical user interfaces, omitting the icons from application suggestions, terminating particular applications after a particular amount of time, and/or permitting access to the particular applications after a predetermined amount of time elapses.

9. The method (200) of claim 8, wherein the adaptations (310a, 310b, 310c) further include one or more of presenting a notification (310a) via graphical user interfaces of the client computing platforms, wherein the notification includes a recommendation and/or a suggestion (310b).

10. The method (200) of claim 8, wherein the psychological parameter values characterize motivations, emotions, emotional intelligence, cultural values, personal values, communication style, personality, psychological wellbeing, and/or learning style.

11. The method (200) of claim 8, wherein identifying the clusters (306a, 306b) of the users includes latent class analysis, hierarchical clustering, K-means clustering, mean-shifting clustering, machine learning, dimensionality reduction, and/or principal component analysis.

12. The method (200) of claim 8, further comprising:
determining behavioral information of the users, wherein identifying the clusters (306a, 306b) of the users is based on the behavioral information,
wherein:
the behavioral information characterizes performances of behavior patterns related to the usage of the applications by the users within the digital application environment, wherein the behavior patterns include individual actions, sets of the actions, ordered sets of the actions, or multiple of the actions performed by the users, wherein the actions include an in-application purchase, an in-application sale, installations of applications, purchases of applications, interactions with users, interactions with content, initiations of particular applications, and/or terminations of the particular applications, and
the application usage information includes screen time, battery usage, Internet usage, location usage, times of the installations, application types of the installations, costs of the installations, times of the initiations, times of the terminations, amount of notifications, notification types of the notifications, cross-application information usage, times of the in-application purchases and the in-application sales, item type of the in-application purchases, the item type of the in-application sales, content types of the content interacted with, interaction types of the interactions, and/or the application types of the applications initiated.

13. The method (200) of claim 8, wherein the application usage information is obtained from individual ones of the applications installed on the client computing platforms, and/or from a recordation application installed on the client computing platforms that aggregates the application usage information from the individual applications.

## Patentansprüche

1. System (100), das zur Anpassung einer digitalen Anwendungsumgebung (312, 322) auf Basis psychologischer Attribute von individuellen Benutzern konfiguriert ist, wobei das System umfasst:
elektronischen Speicher (128), der zum Speichern von Informationen im Zusammenhang mit individuellen Benutzern konfiguriert ist;
einen oder mehrere Prozessoren (130), die durch maschinenlesbare Anweisungen (106) konfiguriert sind zum:
Erhalten (204) von Anwendungsnutzungsinformationen von Client-Computerplattformen (104a, 104b) im Zusammenhang mit Benutzern, wobei die Anwendungsnutzungsinformationen die Benutzung von Anwendungen innerhalb von digitalen Anwendungsumgebungen (312, 322) durch die Benutzer kennzeichnen, wobei die Client-Computerplattformen (104a, 104b) die digitalen Anwendungsumgebungen (312, 322) bereitstellen;
Erhalten (206) von angegebenen Informationen, die von den Benutzern bereitgestellt werden, wobei die angegebenen Informationen Sätze von Antworten auf Fragen einschließen, die sich auf psychologische Attribute beziehen, wobei die individuellen Sätze von Antworten von individuellen der Benutzer derart bereitgestellt werden, dass die Sätze von Antworten einen ersten Satz von Antworten einschließen, der von einem ersten Benutzer bereitgestellt wird;
Bestimmen (208), auf Basis der Sätze von Antworten, von Sätzen von Werten von psychologischen Parametern für psychologische Parameter für die individuellen Benutzer derart, dass ein erster Satz von Werten von psychologischen Parametern für einen ersten Satz von psychologischen Parametern für den ersten Benutzer bestimmt wird;
Identifizieren (210), auf Basis der Anwendungsnutzungsinformationen und der Sätze von Werten von psychologischen Parametern für die individuellen Benutzer, von Clustern (306a, 306b) von Benutzern, die ähnliche Sätze von Werten von psychologischen Parametern aufweisen, wobei die Cluster (306a, 306b) ein erstes Cluster einschließen, das den ersten Benutzer auf Basis des ersten Satzes von Werten von psychologischen Parametern einschließt;
Bestimmen (212) von Anpassungen (310a, 310b, 310c) an die digitalen Anwendungsumgebungen (312, 322), die von den Client-Computerplattformen (104a, 104b) bereitgestellt werden, für die individuellen Benutzer auf Basis der Cluster (306a, 306b) derart, dass eine erste Anpassung an die digitalen Anwendungsumgebungen (312, 322) für das erste Cluster von Benutzern bestimmt wird, das den ersten Benutzer einschließt; und
Übertragen (214) der Anpassungen (310a, 310b, 310c) an die Client-Computerplattformen (104a, 104b) zur Implementierung derart, dass die erste Anpassung an die Client-Computerplattformen (104a, 104b) im Zusammenhang mit dem ersten Cluster von Benutzern zur Implementierung übertragen wird,
**dadurch gekennzeichnet ist, dass**
die Anpassungen (310a, 310b, 310c) das Begrenzen von Zugang zu bestimmten der Anwendungen, das Bewegen von Symbolen, die die Anwendungen nach einer Auswahl auf den graphischen Benutzeroberflächen starten, das Weglassen der Symbole von Anwendungsvorschlägen, das Beenden bestimmter Anwendungen nach einer bestimmten Zeitdauer und/oder das Gewähren von Zugang zu den bestimmten Anwendungen nach Verstreichen einer vorbestimmten Zeitdauer einschließen.

2. System (100) nach Anspruch 1, wobei die Anpassungen (310a, 310b, 310c) weiter Vorlegen einer Benachrichtigung (310a) über graphische Benutzeroberflächen der Client-Computerplattformen (104a, 104b) einschließen, wobei die Benachrichtigung eine Empfehlung und/oder einen Vorschlag (310b) einschließt.

3. System (100) nach Anspruch 1, wobei die Werte von psychologischen Parametern Motivierungen, Gefühle, emotionale Intelligenz, kulturelle Werte, persönliche Werte, Kommunikationsstil, Persönlichkeit, persönliches Wohlbefinden und/oder Lernstil kennzeichnen.

4. System (100) nach Anspruch 1, wobei das Identifizieren der Cluster (306a, 306b) der Benutzer eine latente Klassenanalyse, hierarchisches Clustering, K-Means-Clustering, Mean-Shifting-Clustering, maschinelles Lernen, Dimensionsreduktion und/oder Hauptkomponentenanalyse einschließt.

5. System (100) nach Anspruch 1, wobei der eine oder die mehreren Prozessoren (130) weiter durch maschinenlesbare Anweisungen (106) konfiguriert sind zum:
Bestimmen von Verhaltensinformationen der Benutzer, wobei das Identifizieren der Cluster (306a, 306b) der Benutzer auf den Verhaltensinformationen basiert,
wobei die Verhaltensinformationen Leistungen von Verhaltensmustern im Zusammenhang mit der Nutzung der Anwendungen durch die Benutzer innerhalb der digitalen Anwendungsumgebung (312, 322) kennzeichnen, wobei die Verhaltensmuster individuelle Aktionen, Sätze der Aktionen, geordnete Sätze der Aktionen oder mehrere der Aktionen, die von den Benutzern durchgeführt werden, einschließen, wobei die Aktionen einen Kauf über die Anwendung, einen Verkauf über die Anwendung, Installationen von Anwendungen, Käufe von Anwendungen, Interaktionen mit Benutzern, Interaktionen mit Inhalt, Starten bestimmter Anwendungen und/oder Beenden der bestimmten Anwendungen einschließen.

6. System (100) nach Anspruch 5, wobei die Anwendungsnutzungsinformationen Zeit am Bildschirm, Batterienutzung, Internetnutzung, Standortnutzung, Zeiten der Installationen, Anwendungsarten der Installationen, Kosten der Installationen, Zeiten des Startens, Zeiten des Beendens, Benachrichtigungsmengen, Benachrichtigungsarten der Benachrichtigungen, Informationsnutzung über Anwendungen hinweg, Zeiten der Käufe und der Verkäufe über die Anwendung, Artikelart der Käufe über die Anwendung, Artikelart der Verkäufe über die Anwendung, Inhaltsarten des Inhalts, mit dem interagiert wurde, Interaktionsarten der Interaktionen und/oder Anwendungsarten der gestarteten Anwendungen einschließen.

7. System (100) nach Anspruch 1, wobei die Anwendungsnutzungsinformationen von individuellen der Anwendungen, die auf den Client-Computerplattformen (104a, 104b) installiert sind, und/oder von einer Aufzeichnungsanwendung, die auf den Client-Computerplattformen (104a, 104b) installiert ist und die die Anwendungsnutzungsinformationen von den individuellen Anwendungen aggregiert, erhalten werden.

8. Verfahren (200), das zur Anpassung einer digitalen Anwendungsumgebung (312, 322) auf Basis psychologischer Attribute von individuellen Benutzern konfiguriert ist, wobei das Verfahren umfasst:
Speichern (202), im elektronischen Speicher (128), von Informationen im Zusammenhang mit individuellen Benutzern;
Erhalten (204) von Anwendungsnutzungsinformationen von Client-Computerplattformen (104a, 104b) im Zusammenhang mit Benutzern, wobei die Anwendungsnutzungsinformationen die Benutzung von Anwendungen innerhalb von digitalen Anwendungsumgebungen (312, 322) durch die Benutzer kennzeichnen, wobei die Client-Computerplattformen (104a, 104b) die digitalen Anwendungsumgebungen (312, 322) bereitstellen;
Erhalten (206) von angegebenen Informationen, die von den Benutzern bereitgestellt werden, wobei die angegebenen Informationen Sätze von Antworten auf Fragen einschließen, die sich auf psychologische Attribute beziehen, wobei die individuellen Sätze von Antworten von individuellen der Benutzer derart bereitgestellt werden, dass die Sätze von Antworten einen ersten Satz von Antworten einschließen, der von einem ersten Benutzer bereitgestellt wird;
Bestimmen (208), auf Basis der Anwendungsnutzungsinformationen und der angegebenen Informationen, von Sätzen von Werten von psychologischen Parametern für psychologische Parameter für die individuellen Benutzer derart, dass ein erster Satz von Werten von psychologischen Parametern für einen ersten Satz von psychologischen Parametern für den ersten Benutzer bestimmt wird;
Identifizieren (210), auf Basis der Anwendungsnutzungsinformationen und der Sätze von Werten von psychologischen Parametern für die individuellen Benutzer, von Clustern (306a, 306b) von Benutzern, die ähnliche Sätze von Werten von psychologischen Parametern aufweisen, wobei die Cluster ein erstes Cluster einschließen, das den ersten Benutzer auf Basis des ersten Satzes von Werten von psychologischen Parametern einschließt;
Bestimmen (212) von Anpassungen (310a, 310b, 310c) an die digitalen Anwendungsumgebungen (312, 322), die von den Client-Computerplattformen (104a, 104b) bereitgestellt werden, für die individuellen Benutzer auf Basis der Cluster (306a, 306b) derart, dass eine erste Anpassung an die digitalen Anwendungsumgebungen (312, 322) für das erste Cluster von Benutzern bestimmt wird, das den ersten Benutzer einschließt; und
Übertragen (214) der Anpassungen (310a, 310b, 310c) an die Client-Computerplattformen (104a, 104b) zur Implementierung derart, dass die erste Anpassung an die Client-Computerplattformen (104a, 104b) im Zusammenhang mit dem ersten Cluster von Benutzern zur Implementierung übertragen wird,
**dadurch gekennzeichnet ist, dass**
die Anpassungen (310a, 310b, 310c) das Begrenzen von Zugang zu bestimmten der Anwendungen, das Bewegen von Symbolen, die die Anwendungen nach einer Auswahl auf den graphischen Benutzeroberflächen starten, das Weglassen der Symbole von Anwendungsvorschlägen, das Beenden bestimmter Anwendungen nach einer bestimmten Zeitdauer und/oder das Gewähren von Zugang zu den bestimmten Anwendungen nach Verstreichen einer vorbestimmten Zeitdauer einschließen.

9. Verfahren (200) nach Anspruch 8, wobei die Anpassungen (310a, 310b, 310c) weiter eines oder mehrere von Vorlegen einer Benachrichtigung (310a) über graphische Benutzeroberflächen der Client-Computerplattformen einschließen, wobei die Benachrichtigung eine Empfehlung und/oder einen Vorschlag (310b) einschließt.

10. Verfahren (200) nach Anspruch 8, wobei die Werte von psychologischen Parametern Motivierungen, Gefühle, emotionale Intelligenz, kulturelle Werte, persönliche Werte, Kommunikationsstil, Persönlichkeit, persönliches Wohlbefinden und/oder Lernstil kennzeichnen.

11. Verfahren (200) nach Anspruch 8, wobei das Identifizieren der Cluster (306a, 306b) der Benutzer eine latente Klassenanalyse, hierarchisches Clustering, K-Means-Clustering, Mean-Shifting-Clustering, maschinelles Lernen, Dimensionsreduktion und/oder Hauptkomponentenanalyse einschließt.

12. Verfahren (200) nach Anspruch 8, weiter umfassend:
Bestimmen von Verhaltensinformationen der Benutzer, wobei das Identifizieren der Cluster (306a, 306b) der Benutzer auf den Verhaltensinformationen basiert,
wobei:
die Verhaltensinformationen Leistungen von Verhaltensmustern im Zusammenhang mit der Nutzung der Anwendungen durch die Benutzer innerhalb der digitalen Anwendungsumgebung kennzeichnen, wobei die Verhaltensmuster individuelle Aktionen, Sätze der Aktionen, geordnete Sätze der Aktionen oder mehrere der Aktionen, die von den Benutzern durchgeführt werden, einschließen, wobei die Aktionen einen Kauf über die Anwendung, einen Verkauf über die Anwendung, Installationen von Anwendungen, Käufe von Anwendungen, Interaktionen mit Benutzern, Interaktionen mit Inhalt, Starten bestimmter Anwendungen und/oder Beenden der bestimmten Anwendungen einschließen, und
die Anwendungsnutzungsinformationen Zeit am Bildschirm, Batterienutzung, Internetnutzung, Standortnutzung, Zeiten der Installationen, Anwendungsarten der Installationen, Kosten der Installationen, Zeiten des Startens, Zeiten des Beendens, Benachrichtigungsmengen, Benachrichtigungsarten der Benachrichtigungen, Informationsnutzung über Anwendungen hinweg, Zeiten der Käufe und Verkäufe über die Anwendung, Artikelart der Käufe über die Anwendung, die Artikelart der Verkäufe über die Anwendung, Inhaltsarten des Inhalts, mit dem interagiert wurde, Interaktionsarten der Interaktionen und/oder Anwendungsarten der gestarteten Anwendungen einschließen.

13. Verfahren (200) nach Anspruch 8, wobei die Anwendungsnutzungsinformationen von individuellen der Anwendungen, die auf den Client-Computerplattformen installiert sind, und/oder von einer Aufzeichnungsanwendung, die auf den Client-Computerplattformen installiert ist und die die Anwendungsnutzungsinformationen von den individuellen Anwendungen aggregiert, erhalten werden.

## Revendications

1. Système (100) configuré pour adapter un environnement d'application numérique (312, 322) sur la base de caractéristiques psychologiques d'utilisateurs individuels, le système comprenant :
un stockage électronique (128) configuré pour stocker des informations associées aux utilisateurs individuels ;
un ou plusieurs processeurs (130) configurés par des instructions lisibles par machine (106) pour :
obtenir (204) des informations d'utilisation d'application à partir de plateformes informatiques client (104a, 104b) associées à des utilisateurs, dans lequel les informations d'utilisation d'application caractérisent l'utilisation d'applications au sein d'environnements d'application numériques (312, 322) par les utilisateurs, dans lequel les plateformes informatiques client (104a, 104b) fournissent les environnements d'application numériques (312, 322) ;
obtenir (206) des informations déclarées fournies par les utilisateurs, dans lequel les informations déclarées incluent des ensembles de réponses à des questions qui concernent des caractéristiques psychologiques, dans lequel les ensembles individuels de réponses sont fournis par des utilisateurs individuels parmi les utilisateurs de sorte que les ensembles de réponses incluent un premier ensemble de réponses fournies par un premier utilisateur ;
déterminer (208), sur la base des ensembles de réponses, des ensembles de valeurs de paramètres psychologiques à des paramètres psychologiques pour les utilisateurs individuels de sorte qu'un premier ensemble de valeurs de paramètres psychologiques à un premier ensemble de paramètres psychologiques soit déterminé pour le premier utilisateur ;
identifier (210), sur la base des informations d'utilisation d'application et des ensembles de valeurs de paramètres psychologiques pour les utilisateurs individuels, des groupes (306a, 306b) d'utilisateurs qui présentent des ensembles similaires de valeurs de paramètres psychologiques, les groupes (306a, 306b) incluant un premier groupe qui inclut le premier utilisateur sur la base du premier ensemble de valeurs de paramètres psychologiques ;
déterminer (212) des adaptations (310a, 310b, 310c) aux environnements d'application numériques (312, 322) fournis par les plateformes informatiques client (104a, 104b) pour les utilisateurs individuels sur la base des groupes (306a, 306b) de sorte qu'une première adaptation aux environnements d'application numérique (312, 322) est déterminée pour le premier groupe d'utilisateurs, incluant le premier utilisateur ; et
transmettre (214) les adaptations (310a, 310b, 310c) aux plateformes informatiques client (104a, 104b) pour la mise en œuvre de sorte que la première adaptation soit transmise aux plateformes informatiques client (104a, 104b) associées au premier groupe d'utilisateurs pour la mise en œuvre,
**caractérisé en ce que**
les adaptations (310a, 310b, 310c) incluent la restriction de l'accès à des applications particulières parmi les applications, le déplacement d'icônes qui lancent les applications lors de la sélection sur les interfaces utilisateur graphiques, l'omission des icônes par rapport à des suggestions d'application, la terminaison d'applications particulières après une quantité de temps particulière, et/ou l'autorisation d'accès aux applications particulières après qu'une quantité de temps prédéterminée expire.

2. Système (100) selon la revendication 1, dans lequel les adaptations (310a, 310b, 310c) incluent en outre la présentation d'une notification (310a) via des interfaces utilisateur graphiques des plateformes informatiques client (104a, 104b), dans lequel la notification inclut une recommandation et/ou une suggestion (310b).

3. Système (100) selon la revendication 1, dans lequel les valeurs de paramètres psychologiques caractérisent des motivations, des émotions, une intelligence émotionnelle, des valeurs culturelles, des valeurs personnelles, un style de communication, une personnalité, un bien-être psychologique, et/ou un style d'apprentissage.

4. Système (100) selon la revendication 1, dans lequel l'identification des groupes (306a, 306b) des utilisateurs inclut une analyse de classe latente, un groupement hiérarchique, un regroupement de moyennes K, un regroupement de déplacement de moyenne, un apprentissage automatique, une réduction de dimensionnalité et/ou une analyse de composants principaux.

5. Système (100) selon la revendication 1, dans lequel les un ou plusieurs processeurs (130) sont en outre configurés par des instructions lisibles par machine (106) pour :
déterminer des informations comportementales des utilisateurs, dans lequel l'identification des groupes (306a, 306b) des utilisateurs est basée sur les informations comportementales,
dans lequel les informations comportementales caractérisent des performances de modèles de comportement liés à l'utilisation des applications par les utilisateurs au sein de l'environnement d'application numérique (312, 322), dans lequel les modèles de comportement incluent des actions individuelles, des ensembles d'actions, des ensembles ordonnés des actions, ou plusieurs des actions réalisées par les utilisateurs, dans lequel les actions incluent un achat dans l'application, une vente dans l'application, des installations d'applications, des achats d'applications, des interactions avec des utilisateurs, des interactions avec un contenu, des lancements d'applications particulières et/ou des terminaisons des applications particulières.

6. Système (100) selon la revendication 5, dans lequel les informations d'utilisation de l'application incluent un temps d'écran, une utilisation de batterie, une utilisation d'Internet, une utilisation d'emplacement, des temps des installations, des types d'application des installations, des coûts des installations, des temps des lancements, des temps des terminaisons, une quantité de notifications, des types de notification des notifications, une utilisation d'informations inter-applications, des temps des achats dans l'application et des ventes dans l'application, un type d'article des achats dans l'application, le type d'article des ventes dans l'application, des types de contenu du contenu sujet à une interaction, des types d'interaction des interactions, et/ou des types d'applications des applications lancées.

7. Système (100) selon la revendication 1, dans lequel les informations d'utilisation d'application sont obtenues à partir d'applications individuelles parmi les applications installées sur les plateformes informatiques client (104a, 104b), et/ou à partir d'une application d'enregistrement installée sur les plateformes informatiques client (104a, 104b) qui agrège les informations d'utilisation d'application à partir des applications individuelles.

8. Procédé (200) d'adaptation d'un environnement d'application numérique (312, 322) sur la base de caractéristiques psychologiques d'utilisateurs individuels, le procédé comprenant :
le stockage (202), dans un stockage électronique (128), d'informations associées aux utilisateurs individuels ;
l'obtention (204) d'informations d'utilisation d'application à partir de plateformes informatiques client (104a, 104b) associées à des utilisateurs, dans lequel les informations d'utilisation d'application caractérisent l'utilisation d'applications au sein d'environnements d'application numériques (312, 322) par les utilisateurs, dans lequel les plateformes informatiques client (104a, 104b) fournissent les environnements d'application numériques (312, 322) ;
l'obtention (206) d'informations déclarées fournies par les utilisateurs, dans lequel les informations déclarées incluent des ensembles de réponses à des questions qui concernent des caractéristiques psychologiques, dans lequel les ensembles individuels de réponses sont fournis par des utilisateurs individuels parmi les utilisateurs de sorte que les ensembles de réponses incluent un premier ensemble de réponses fournies par un premier utilisateur ;
la détermination (208), sur la base des informations d'utilisation d'application et des informations déclarées, d'ensembles de valeurs de paramètres psychologiques à des paramètres psychologiques pour les utilisateurs individuels de sorte qu'un premier ensemble de valeurs de paramètres psychologiques à un premier ensemble de paramètres psychologiques soit déterminé pour le premier utilisateur ;
l'identification (210), sur la base des informations d'utilisation d'application et des ensembles de valeurs de paramètres psychologiques pour les utilisateurs individuels, de groupes (306a, 306b) d'utilisateurs qui présentent des ensembles similaires de valeurs de paramètres psychologiques, les groupes incluant un premier groupe qui inclut le premier utilisateur sur la base du premier ensemble de valeurs de paramètres psychologiques ;
la détermination (212) d'adaptations (310a, 310b, 310c) aux environnements d'application numériques (312, 322) fournis par les plateformes informatiques client (104a, 104b) pour les utilisateurs individuels sur la base des groupes (306a, 306b) de sorte qu'une première adaptation aux environnements d'application numérique (312, 322) est déterminée pour le premier groupe d'utilisateurs, incluant le premier utilisateur ; et
la transmission (214) des adaptations (310a, 310b, 310c) aux plateformes informatiques client (104a, 104b) pour la mise en œuvre de sorte que la première adaptation soit transmise aux plateformes informatiques client (104a, 104b) associées au premier groupe d'utilisateurs pour la mise en œuvre,
**caractérisé en ce que**
les adaptations (310a, 310b, 310c) incluent la restriction de l'accès à des applications particulières parmi les applications, le déplacement d'icônes qui lancent les applications lors de la sélection sur les interfaces utilisateur graphiques, l'omission des icônes par rapport à des suggestions d'application, la terminaison d'applications particulières après une quantité de temps particulière, et/ou l'autorisation d'accès aux applications particulières après qu'une quantité de temps prédéterminée expire.

9. Procédé (200) selon la revendication 8, dans lequel les adaptations (310a, 310b, 310c) incluent en outre une ou plusieurs parmi la présentation d'une notification (310a) via des interfaces utilisateur graphiques des plateformes informatiques client (104a, 104b), dans lequel la notification inclut une recommandation et/ou une suggestion (310b).

10. Procédé (200) selon la revendication 8, dans lequel les valeurs de paramètres psychologiques caractérisent des motivations, des émotions, une intelligence émotionnelle, des valeurs culturelles, des valeurs personnelles, un style de communication, une personnalité, un bien-être psychologique, et/ou un style d'apprentissage.

11. Procédé (200) selon la revendication 8, dans lequel l'identification des groupes (306a, 306b) des utilisateurs inclut une analyse de classe latente, un groupement hiérarchique, un regroupement de moyennes K, un regroupement de déplacement de moyenne, un apprentissage automatique, une réduction de dimensionnalité et/ou une analyse de composants principaux.

12. Procédé (200) selon la revendication 8, comprenant en outre :
la détermination d'informations comportementales des utilisateurs, dans lequel l'identification des groupes (306a, 306b) des utilisateurs est basée sur les informations comportementales,
dans lequel :
les informations comportementales caractérisent des performances de modèles de comportement liés à l'utilisation des applications par les utilisateurs au sein de l'environnement d'application numérique, dans lequel les modèles de comportement incluent des actions individuelles, des ensembles des actions, des ensembles ordonnés des actions ou plusieurs des actions réalisées par les utilisateurs, dans lequel les actions incluent un achat dans l'application, une vente dans l'application, des installations d'applications, des achats d'applications, des interactions avec des utilisateurs, des interactions avec un contenu, des lancements d'applications particulières et/ou des terminaisons des applications particulières, et
les informations d'utilisation de l'application incluent un temps d'écran, une utilisation de batterie, une utilisation d'Internet, une utilisation d'emplacement, des temps des installations, des types d'application des installations, des coûts des installations, des temps des lancements, des temps des terminaisons, une quantité de notifications, des types de notification des notifications, une utilisation d'informations inter-applications, des temps des achats dans l'application et des ventes dans l'application, un type d'article des achats dans l'application, le type d'article des ventes dans l'application, des types de contenu du contenu sujet à une interaction, des types d'interaction des interactions, et/ou des types d'applications des applications lancées.

13. Procédé (200) selon la revendication 8, dans lequel les informations d'utilisation d'application sont obtenues à partir d'applications individuelles parmi les applications installées sur les plateformes informatiques client, et/ou à partir d'une application d'enregistrement installée sur les plateformes informatiques client qui agrège les informations d'utilisation d'application à partir des applications individuelles.
